# EUROPEAN PATENT APPLICATION

(11) **EP 3 398 516 A1**
(43) Date of publication of application: **07.11.2018**
(21) Application number: 17747778.3
(22) Date of filing: 03.02.2017
(51) Int. Cl.: A61B 6/04, A61B 5/055, A61B 5/00

(54) **MEDICAL TABLE AND MEDICAL IMAGING APPARATUS INCLUDING SAME**

(30) Priority: 03.02.2016 KR 20160013335
(71) Applicant: Samsung Electronics Co., Ltd., Suwon-si, Gyeonggi-do 16677 (KR)
(72) Inventor: DAVE, Vikram Keertikumar, Suwon-si Gyeonggi-do 16543 (KR)
(74) Representative: Grootscholten, Johannes A.M.
(86) International application number: PCT/KR2017/001193
(87) International publication number: WO 2017/135733

(57) **Abstract**

Provided is a medical table and a medical imaging apparatus having the same in which a driving force capable of lifting or lowering the medical table can be efficiently transmitted. The medical imaging apparatus includes a main body at which an image of a patient is taken, and a medical table on which the patient is positioned. The medical table includes a lifting device provided to be extended or shortened in an upward and downward direction, a fixing portion supported by the lifting device, and a transport portion provided to be slidable in a forward and rearward direction while being seated on the fixing portion.

## Description

### [Technical Field]

The present disclosure relates to a medical table having an improved structure and a medical imaging apparatus having the same.

### [Background Art]

A medical imaging apparatus is a device to acquire patient information and provide an image for diagnosing various diseases. The medical imaging apparatus includes an ultrasonic diagnostic apparatus, a radiographic apparatus, a magnetic resonance imaging apparatus, a medical diagnostic apparatus and the like.

The radiographic apparatus is an image system for acquiring an image of a substance, structure, or tissue inside a human body by irradiating X-rays to the whole or a part of the human body. The radiographic apparatus can be used in a medical image system to detect abnormalities such as lesions inside the human body.

The magnetic resonance imaging apparatus is relatively free of an imaging condition, provides excellent contrast in soft tissues, and provides various diagnostic information images, thus occupying an important position in the field of diagnosis using a medical image. When a constant frequency and energy are supplied in a state in which a constant electromagnetic field is applied to an atomic nucleus, a resonance phenomenon occurs and energy is released. The magnetic resonance imaging apparatus is an imaging diagnostic device to diagnose the inside of the human body by converting that energy into a signal.

The radiographic apparatus and the magnetic resonance imaging apparatus may include a gantry having an opening formed at the center thereof. The gantry may be provided in a substantially cylindrical shape. An X-ray generator and an X-ray detector may be positioned to face each other at the gantry provided in the radiographic apparatus. The gantry provided in the magnetic resonance imaging apparatus may be provided with a coil assembly.

The patient to be imaged can be placed on a table and moved into the gantry through the opening formed at the gantry. The table may be provided to be lifted or lowered. A patient with poor mobility can be placed on the table after the table is lowered. After the patient is placed on the table, the table can be lifted to match the height of the opening formed at the gantry.

### [Disclosure]

### [Technical Problem]

It is an aspect of the present disclosure to provide a medical table and a medical imaging apparatus having the same in which a driving force capable of lifting or lowering the medical table can be efficiently transmitted.

It is another aspect of the present disclosure to provide a medical table and a medical imaging apparatus having the same in which an accurate image can be acquired.

### [Technical Solution]

In accordance with an aspect of the present disclosure, a medical imaging apparatus may include a main body at which an image of a patient is taken and a medical table on which the patient is positioned. The medical table may include a lifting device provided to be extended or shortened, a fixing portion supported by the lifting device, and a transport portion provided to be slidable in a forward and rearward direction while being seated on the fixing portion.

The lifting device may include a cylindrical portion provided to support a bottom surface of the fixing portion and to extend or shorten in an upward and downward direction, and a driving source transmitting a driving force to extend or shorten the cylindrical portion.

The cylindrical portion may be configured to extend while being rotated in one direction by the driving source, and to shorten while being rotated in the other direction by the driving source.

The fixing portion and the transport portion may be lifted when the cylindrical portion extends, and the fixing portion and the transport portion are lowered when the cylindrical portion shortens.

One side of the lifting device may be mounted on a base, and the other side of the lifting device may support a bottom surface of the fixing portion.

The fixing portion may be supported by a plurality of link portions having at least one side which is slidably provided.

The lifting device may be extended or shortened in a direction perpendicular to the bottom surface.

The lifting device may be positioned between the plurality of link portions.

The plurality of link portions may include a first link having one side mounted on the bottom surface of the fixing portion and the other side mounted on the base, and a second link having one side mounted on the base and the other side mounted on the bottom surface of the fixing portion.

The first link and the second link may be pivotably coupled to a central shaft, the first link may be pivotably provided with respect to the fixing portion, and the second link may be pivotably provided with respect to the base.

A first rail may be provided on the bottom surface of the fixing portion to guide one side of the first link to slide, and a second rail may be provided on the base to guide one side of the second link to slide.

The main body may be provided with an opening, and the transport portion may slide into the main body through the opening.

A weight detection sensor for detecting the weight of the patient positioned on the transport portion may be provided between the lifting device and the fixing portion.

The main body may be tilted according to information detected by the weight detection sensor.

In accordance with an aspect of the present disclosure, a medical table may position a patient in an opening formed at a gantry. The medical table may include a transport portion on which the patient is positioned and introduced into the opening, a fixing portion to which the transport portion is slidably coupled, a lifting device provided to lift or lower the fixing portion by extending or shortening, and a link portion supporting a bottom surface of the fixing portion and having a plurality of links pivotably disposed about a central shaft.

The lifting device may be extended or shortened in a direction perpendicular to a bottom surface.

In accordance with an aspect of the present disclosure, the medical table may further include a base spaced apart from the fixing portion. The lifting device and the link portion may be positioned between the fixing portion and the base.

The lifting device may include a cylindrical portion extended or shortened by a driving source.

The lifting device may transmit a force to the fixing portion in the same direction as a movement direction of the fixing portion.

A weight detection sensor may be provided between the lifting device and the fixing portion to detect the weight of the patient positioned on the transport portion.

### [Advantageous Effects]

According to one embodiment of the present disclosure, a volume of a lifting device mounted on a medical table can be reduced, and the manufacturing cost can be reduced.

In addition, an accurate image of a patient can be obtained by compensating for sagging of a transport portion depending on the weight of the patient positioned on the medical table.

### [Description of Drawings]

FIG. 1 is a view showing a medical imaging apparatus according to an embodiment of the present disclosure;
FIG. 2 is a view showing a medical table according to an embodiment of the present disclosure;
FIG. 3 is a view showing that the medical table, according to an embodiment of the present disclosure, is lowered;
FIG. 4 is a view showing that a patient is placed on the medical table according to an embodiment of the present disclosure;
FIG. 5 is a view showing that a transport portion of the medical table, according to an embodiment of the present disclosure, is positioned inside a gantry; and
FIG. 6 is a view showing that the gantry, according to an embodiment of the present disclosure, is tilted.

### [Modes of the Invention]

Hereinafter, a medical table and a medical imaging apparatus including the medical table according to an embodiment of the present disclosure will be described in detail with reference to drawings.

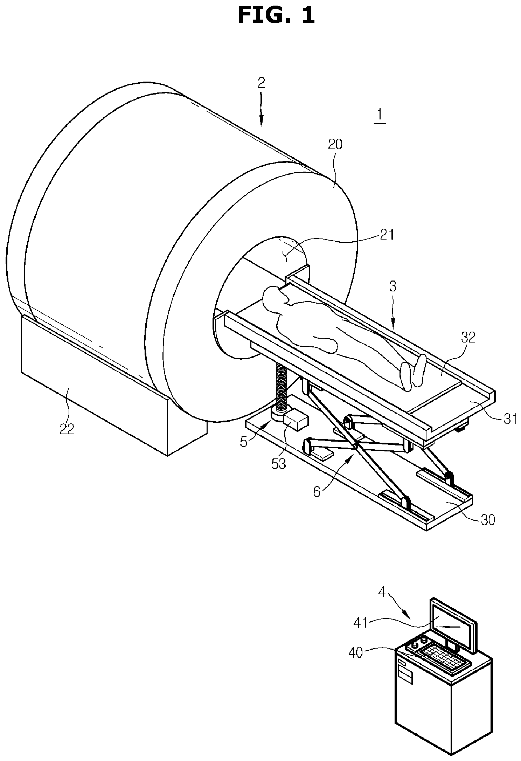

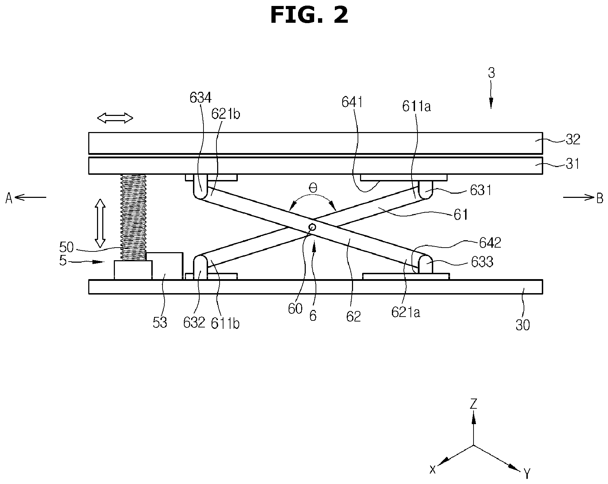

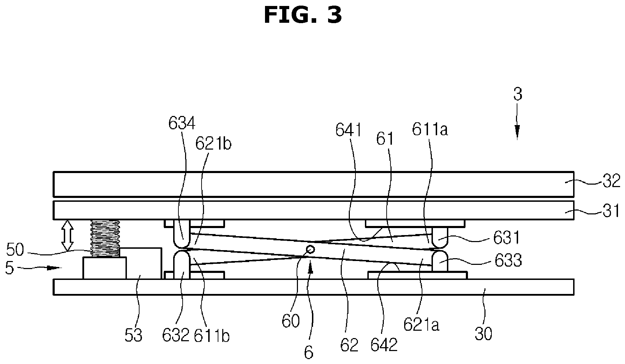

FIG. 1 is a view showing a medical imaging apparatus according to an embodiment of the present disclosure, and FIG. 2 is a view showing a medical table according to an embodiment of the present disclosure. FIG. 3 is a view showing that the medical table, according to an embodiment of the present disclosure, is lowered.

As shown in FIGS. 1 to 3, a medical imaging apparatus 1 according to an embodiment of the present disclosure is an apparatus to obtain an image including information on a living tissue of a patient. The medical imaging apparatus 1 may include a main body 2 at which an image of the patient is taken, and a medical table 3 on which the patient is positioned. The medical imaging apparatus 1 may be operated by an external control device 4.

Hereinafter, a part of a body of the patient which is a subject of the imaging is referred to as an object.

The medical imaging apparatus 1 may be an X-ray imaging apparatus, a computed tomography, or the like that images an image of the inside of a human body by using an X-ray, or may be a magnetic resonance imaging apparatus that acquires the image of the human body by using a magnetic resonance phenomenon. However, the kind of the medical imaging apparatus 1 is not limited to those described above.

The main body 2 may include a gantry 20 having a cylindrical shape, and a stand 22 to support the gantry 20. An opening 21 may be formed at the gantry 20. The patient can be inserted into the opening 21. An image of the object can be taken with the patient inserted into the opening 21.

An X-ray generator and an X-ray detector may be positioned in the gantry 20 to be opposed to each other so that tomography is performed on the object. In addition, a coil assembly may be provided in the gantry 20 to obtain a magnetic resonance image.

The patient may be inserted into the opening 21 while being positioned on the medical table 3. The medical table 3 may include a fixing portion 31 and a transport portion 32. The transport portion 32 may be positioned above the fixing portion 31. The transport portion 32 may be slidably inserted into the opening 21 formed at the gantry 20. The transport portion 32 may be made of a material through which an X-ray or a magnetic field can pass to enable 3D stereoscopic imaging of the object positioned on the transport portion 32.

When the patient is positioned on the transport portion 32, the transport portion 32 may be slid forward and inserted into the opening 21. When taking the image is completed, the transport portion 32 may be slid reward and come out of the opening 21.

The transport portion 32 may be provided to be lifted or lowered. When the transport portion 32 is fixed at a height at which the transport portion 32 can be inserted into the opening 21 of the gantry 20, it may be difficult for the patient with poor mobility to move up the transport portion 32 himself or herself or to be placed on the transport portion 32 by others. Therefore, the transport portion 32 may be provided to be lifted or lowered so that the transport portion 32 is lowered and positioned at a lower height.

A base 30 may be provided under the fixing portion 31. The base 30 may be seated on a bottom surface to be spaced apart from the fixing portion 31 by a predetermined distance.

A lifting device 5 may be provided between the base 30 and the fixing portion 31. The lifting device 5 may be provided to support a bottom surface of the fixing portion 31 and to be extended or shortened. The lifting device 5 may be provided to be extended or shortened in a vertical direction from the bottom surface on which the base 30 is positioned.

When a direction in which the fixing portion 31 is lifted or lowered is referred to as a z-axis direction, the lifting device 5 may be provided to be extended or lowered in the z-axis direction. When the lifting device 5 is extended in the z-axis direction, the fixing portion 31 may be lifted. When the lifting device 5 is shortened in the z-axis direction, the fixing portion 31 may be lowered.

As described above, since the lifting device 5 is provided to be extended or shortened in the z-axis direction to lift or lower the fixing portion 31, it is possible to lift or lower the fixing portion with a small force than that of a conventional case. Since the fixing portion 31 can be lifted or lowered by the small force than that of the conventional case, the lifting device 5 may be driven by a driving source having a small output. Therefore, the size of the driving source required for the lifting device 5 can be smaller, and the cost required for implementing the lifting device 5 can be reduced.

The lifting device 5 may be a device provided to be capable of extending or shortening in one direction by a driving source 53. The lifting device 5 may include a conventional lifting device.

For example, the lifting device 5 may include a metal panel unit in which a metal panel having a predetermined width is wound a plurality of times. The metal panel unit may be positioned on the base 30. One end of the metal panel may be mounted on the bottom surface of the fixing portion 31.

The metal panel unit may be provided to rotate in one direction or another direction by the driving source. When the metal panel unit is rotated in one direction, the metal panel wound on the base 30 may be unwound to form a cylindrical portion 50. As the metal panel unit rotates in one direction, a length of the cylindrical portion 50 can be longer. When the length of the cylindrical portion 50 becomes longer, the fixing portion 31 may be pushed up. Thereby, the fixing portion 31 can be lifted.

The metal panel wound on the base 30 may be coupled to one side of the adjacent metal panel while being unwound. For example, a plurality of coupling protrusions may be formed at one side edge of the metal panel, and a coupling hole may be formed at the other edge of the metal panel into which the coupling protrusions may be inserted. The metal panel may be spirally unwound. As the metal panel is unwound, the coupling protrusions provided at one side edge of the metal panel may be inserted into the adjacent coupling hole. As a result, the length of the cylindrical portion 50 formed by the metal panel may become longer, and the fixing portion 31 may be lifted.

Conversely, when the metal panel unit is rotated in another direction, the coupling protrusions may be come out of the coupling hole and be wound. As the metal panel unit is rotated in another direction, the length of the cylindrical portion 50 formed by the metal panel may become shorter, and the fixing portion 31 may be lowered.

As described above, the metal panel can lift or lower the fixing portion 31 while rotating in one direction or another direction. A configuration of the lifting device 5 may be similar to that disclosed in the previously disclosed U.S. Patent No. 4,875,660.

A link portion 6 may be further provided between the base 30 and the fixing portion 31. One side of the link portion 6 may be coupled to the base 30 and the other side of the link portion 6 may be coupled to the fixing portion 31 to support the fixing portion 31. The link portion 6 may support the fixing portion 31 together with the lifting device 5.

The link portion 6 may include a first link 61 and a second link 62 disposed to be crossed. The first link 61 and the second link 62 may be pivotably disposed about a central shaft 60. When the fixing portion 31 is lifted or lowered by the lifting device 5, an angle θ between the first link 61 and the second link 62 may be variable. When the fixing portion 31 is lifted, the angle θ between the first link 61 and the second link 62 becomes smaller. When the fixing portion 31 is lowered, the angle θ between the first link 61 and the second link 62 becomes larger.

One end 611a of the first link 61 may be mounted on the bottom surface of the fixing portion 31. The one end 611a of the first link 61 may be mounted at a first hinge 631 mounted on the bottom surface of the fixing portion 31. The one end 611a of the first link 61 may be pivotably mounted about the first hinge 631.

The first hinge 631 may be slidably provided along a first rail 641 provided on the bottom surface of the fixing portion 31. When the fixing portion 31 is lifted or lowered by the lifting device 5, the first hinge 631 may slide in one direction or the other direction along the first rail 641.

Other end 611b of the first link 61 may be mounted on an upper surface of the base 30. The other end 611b of the first link 61 may be mounted at a second hinge 632 mounted on the upper surface of the base 30. The other end 611b of the first link 61 may be pivotably mounted about the second hinge 632.

The second hinge 632 may be fixed on the upper surface of the base 30 not to move. The first hinge 631 and the second hinge 632 may be positioned on a straight line parallel to a direction in which the transport portion 32 slides.

The second link 62 may be provided to intersect the first link 61. The second link 62 and the first link 61 may be pivotably provided about the central shaft 60. One end 621a of the second link 62 may be mounted on the upper surface of the base 30. The one end 621a of the second link 62 may be mounted at a third hinge 633 mounted on the upper surface of the base 30. The one end 621a of the second link 62 may be pivotably provided about the third hinge 633.

The third hinge 633 may be slidable along a second rail 642 provided on the upper surface of the base 30. When the fixing portion 31 is lifted or lowered by the lifting device 5, the third hinge 633 may slide in one direction or the other direction along the second rail 642.

Other end 621b of the second link 62 may be mounted on the bottom surface of the fixing portion 31. The other end 621b of the second link 62 may be pivotably mounted at a fourth hinge 634 mounted on the bottom surface of the fixing portion 31. The fourth hinge 634 may be fixed on the bottom surface of the fixing portion 31 not to move. However, the other end 621b of the second link 62 may be pivoted about the fourth hinge 634 as the fixing portion 31 is lifted or lowered.

The third hinge 633 and the fourth hinge 634 may be positioned on a straight line extending in a direction parallel to a direction in which the transport portion 32 slides.

It is assumed that a direction in which the transport portion 32 slides and protrudes from the fixing portion 31 is referred to as a front side, and a direction in which the transport portion 32 slides to return is referred to as a rear side. At this time, the first hinge 631 and the third hinge 633 which are slidably provided may be positioned at the rear side, and the second hinge 632 fixed on the upper surface of the base 30 and the fourth hinge 634 fixed on the bottom surface of the fixing portion 31 may be positioned at the front side.

The lifting device 5 may be positioned in front of the link portion 6. The lifting device 5 may be positioned to overlap with the link portion 6. In detail, a plurality of the link portions 6 may be provided at both lateral sides of the medical table 3, and the lifting device 5 may be disposed between the plurality of the link portions 6 provided at the both lateral sides of the medical table 3.

As described above, by positioning the lifting device 5 and the link portion 6 in a forward and rearward direction or positioning the lifting device 5 and the link portion 6 to be overlapped with each other, it is possible to prevent the lateral width of the medical table 3 from widening. As a result, the medical table 3 can be slimly realized.

The base 30 or the fixing portion 31 may be provided with a brake device (not shown) capable of braking a movement of the first hinge 631 or the third hinge 633 slidably provided along the rails 641 and 642. When the fixing portion 31 is positioned at a specific height by the lifting device 5, the first hinge 631 or the third hinge 633 may be braked by the brake device so that a sliding operation of the first hinge 631 or the third hinge 633 can be stopped. Whereby the fixing portion 31 may be supported by the link portion 6 while being positioned at the specific height.

In a case of the present disclosure, the lifting device 5 may be provided to move only in the direction in which the fixing portion 5 is lifted or lowered. According to the present disclosure, the fixing portion 31 may be lifted or lowered with a small force than that of a conventional case.

In the conventional case, a link portion supporting a fixing portion was provided, and a driving force capable of lifting or lowering the fixing portion was transmitted through one side of the link portion. The driving force transmitted through the link portion was transmitted in a forward and rearward direction or in an extending direction of the link portion. A part of the force transmitted through the link portion was transmitted in a direction in which one side of the link portion was slid and the remaining force was transmitted in a direction in which the fixing portion was lifted or lowered.

Since only a part of the force transmitted from the driving source was used to lift or lower the fixing portion, an output of the driving source had to be provided larger than an output required to lift or lower the fixing portion. Thus, conventionally, the fixing portion was lifted or lowered by the driving source having a large output and a large volume. Therefore, the manufacturing cost required for the lifting device of the medical table was increased, and a volume of the lifting device could be increased more than necessary.

However, in the present disclosure, since the lifting device 5 acts on a force in the direction in which the fixing portion 31 is lifted or lowered, the fixing portion 31 can be lifted or lowered by the driving source having a smaller output than the conventional one. Therefore, the cost required for manufacturing the lifting device can be reduced, and the lifting device can be downsized.

The medical imaging apparatus 1 may further include the control device 4. The control device 4 may be provided outside and may be operated by an operator. An operation of the main body 2 and the medical table 3 may be controlled by the control device 4. The control device 4 may include an operating portion 40 and a display portion 41. The operator may control the operation of the main body 2 and the medical table 3 by operating the operating portion 40. The display portion 41 may display information on a state of the main body 20 and the medical table 3, and images acquired for the object.

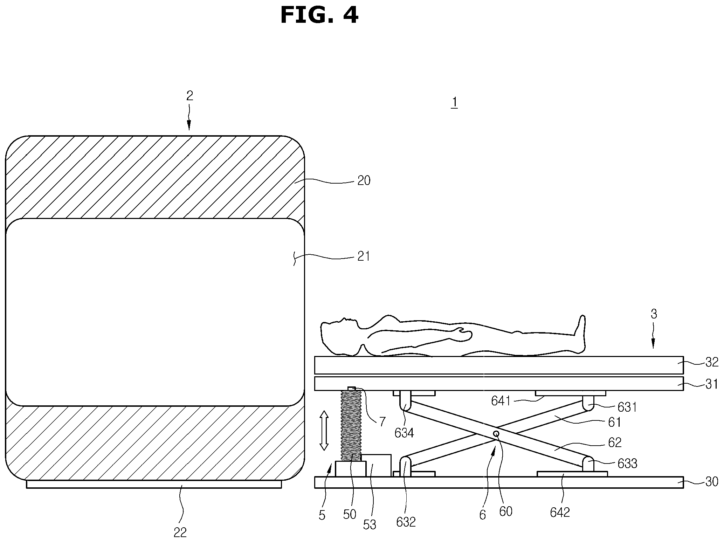

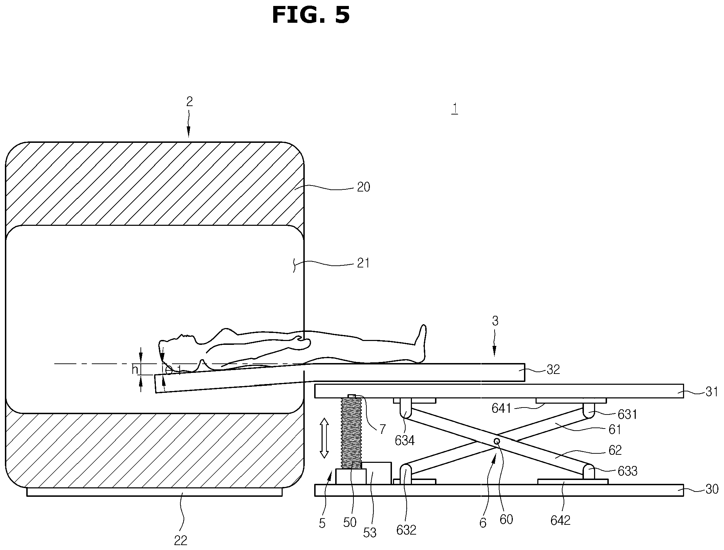

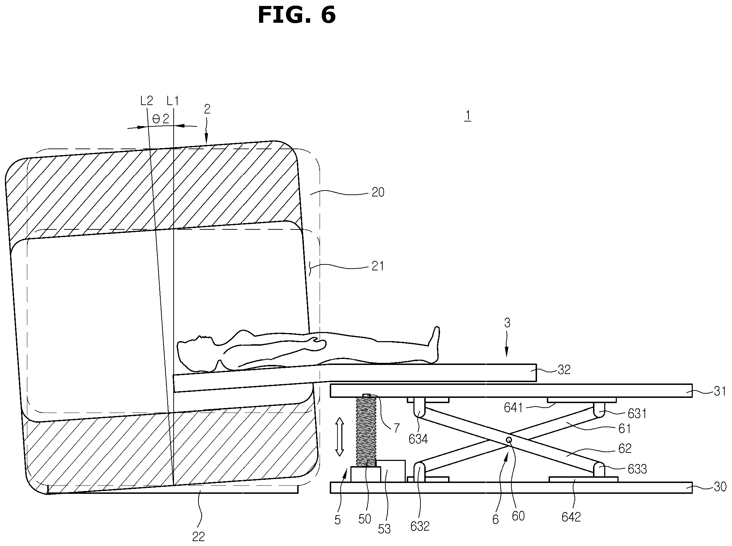

FIG. 4 is a view showing that a patient is placed on the medical table according to an embodiment of the present disclosure, and FIG. 5 is a view showing that a transport portion of the medical table, according to an embodiment of the present disclosure, is positioned inside a gantry. FIG. 6 is a view showing that the gantry, according to an embodiment of the present disclosure, is tilted.

As shown in FIGS. 4 to 6, the patient may be positioned on the transport portion 32 of the medical table 3 according to one embodiment of the present disclosure, and may be moved into the gantry 2 for taking an image. The transport portion 32 may slide forward and move into the gantry 2.

The operator may lower the transport portion 32 by operating the control device 41 when positioning the patient with poor mobility on the transport portion 32. The patient may be easily positioned on the transport portion 32 which is lowered and positioned at a lower height. After the patient is positioned on the transport portion 32, the operator can operate the control device 41 to lift the transport portion 32. The transport portion 32 may be lifted to a position where the transport portion 32 is inserted into the opening 21 provided at the gantry 20.

The transport portion 32 may slide toward the opening 21 formed at the gantry 20 while the patient is positioned thereon. The transport portion 32 may slide and protrude from the fixing portion 31. The transport portion 32 protruding from the fixing portion 31 may be bent and sagged downward by the weight of the patient positioned thereon. An angle between an upper surface of the transport portion 32 positioning on an upper side of the fixing portion 31 in front of the fixing portion 31 and the transport portion 32 protruding from the fixing portion 31 may be defined as θ1.

The gantry 20 may be provided so that an accurate image of the object can be obtained when the patient is positioned in the opening 21 in parallel with a straight line passing through the center of the opening 21. That is, when the transport portion 32 is slid in a direction parallel to the fixing portion 31 to position the patient in the opening 21, the accurate image can be obtained by an imaging device provided at the gantry 20. However, when the transport portion 32 positioned inside the opening 21 is not positioned in parallel with the fixing portion 31 and is bent at the predetermined angle θ1, image information on the object can be acquired incorrectly.

In a case of the present disclosure, the gantry 20 may be tilted by a predetermined angle θ2 to compensate the angle θ1 at which the transport portion 32 is inclined. The angle θ2 at which the gantry 20 is tilted may be an angle formed by a straight line L1 passing through the center of the gantry 20 before tilting and a straight line L2 passing through the center of the gantry 20 after being tilted.

The angle θ2 at which the gantry 20 is tilted and the angle θ1 at which the transport portion 32 is inclined may be the same. That is, if the transport portion 32 is inclined in a counter clockwise direction by the angle θ1, the gantry 20 may also be tilted by the angle θ1 in the counterclockwise direction. The angle at which the transport portion 32 is inclined and the angle at which the gantry 20 is tilted may be different from each other.

As described above, the gantry 20 is tilted according to the angle at which the transport portion 32 is inclined, so that the accurate image of the object can be obtained.

An angle at which the gantry 20 is tilted by the control device 4 may be controlled. The control device 4 may control a tilting angle of the gantry 20 according to the angle at which the transport portion 32 is inclined, so that the accurate image of the patient can be obtained.

A weight detection sensor 7 may be provided between the fixing portion 31 and the lifting device 5. The weight detection sensor 7 may be mounted on the bottom surface of the fixing portion 31. The weight detection sensor 7 may detect the weight of the patient positioned on the transport portion 32 and may transmit the detected weight to the control device 4. The control device 4 may be provided with a predetermined degree of bending of the transport portion 32 in accordance with a force applied to the transport portion 32. The control device 4 receiving the information on the weight of the patient may calculate a degree of bending of the transport portion 32 based on the inputted data to tilt the gantry 20.

The weight detection sensor 7 may be mounted on a front bottom surface of the fixing portion 31. The weight detection sensor 7 may be positioned in front of the fixing portion 31 so that the weight of the patient to be transported and positioned on the transport portion 32 can be accurately measured. The weight detection sensor 7 may be positioned between the lifting device 5 and the fixing portion 31, or may be mounted on the bottom surface of the fixing portion 31.

In this way, the weight of the patient positioned on the transport portion 32 may be measured by the weight detection sensor 7, and the degree of bending of the transport portion 32 according to the measured patient's weight can be calculated. The control device 4 may tilt the gantry 20 according to the degree of bending of the transport portion 32 so that the patient is positioned parallel to the straight line passing through the center of the opening 21. Thus, even if the transport portion 32 is bent by the weight of the patient, the accurate image can be obtained.

In the above description, the gantry 20 is tilted when the transport portion 32 is bent. However, when it is necessary to obtain an accurate image of the object, an operator can operate the control device 4 to tilt the gantry 20 at a predetermined angle.

A structure for tilting the gantry 20 may be an existing structure.

According to the above, the medical table may be provided to be lifted or lowered in an upward and downward direction, so that even a patient with poor mobility can be easily positioned on the medical table by lowering the medical table. In addition, since the lifting device is provided to apply force to a bottom surface of the medical table in the upward and downward direction, a magnitude of a force required to lift or lower the medical table compared to the conventional case. A size of the driving source for driving the lifting device can be reduced by providing a small force required for lifting or lowering the medical table. Therefore, the lifting device can be slimly realized.

The lifting device may support the medical table together with the link portion. The lifting device may be positioned between the plurality of link portions, or may be positioned in front of the plurality of link portions. It is possible to prevent a width of the medical table from being widened by the lifting device as the lifting device is positioned in front of the plurality of link portions.

The medical table may be provided with the weight detection sensor to detect the weight of the patient positioned on the medical table. The weight of the patient can be detected by the weight detection sensor. The control device can calculate the degree of bending of the transport portion according to the weight of the patient, and can control the gantry to be tilted at a predetermined angle according to the degree of bending of the transport portion. This makes it possible to obtain an accurate image of the object.

## Claims

1. A medical imaging apparatus comprising:
a main body at which an image of a patient is taken; and
a medical table on which the patient is positioned,
wherein the medical table comprises:
a lifting device provided to be extended or shortened;
a fixing portion supported by the lifting device; and
a transport portion provided to be slidable in a forward and rearward direction while being seated on the fixing portion.

2. The medical imaging apparatus according to claim 1, wherein the lifting device comprises:
a cylindrical portion provided to support a bottom surface of the fixing portion and to extend or shorten in an upward and downward direction; and
a driving source transmitting a driving force to extend or shorten the cylindrical portion.

3. The medical imaging apparatus according to claim 1, wherein the cylindrical portion is configured to extend while being rotated in one direction by the driving source, and to shorten while being rotated in the other direction by the driving source.

4. The medical imaging apparatus according to claim 3, wherein the fixing portion and the transport portion are lifted when the cylindrical portion extends, and the fixing portion and the transport portion are lowered when the cylindrical portion shortens.

5. The medical imaging apparatus according to claim 1, wherein one side of the lifting device is mounted on a base, and the other side of the lifting device supports a bottom surface of the fixing portion.

6. The medical imaging apparatus according to claim 5, wherein the fixing portion is supported by a plurality of link portions having at least one side which is slidably provided.

7. The medical imaging apparatus according to claim 1, wherein the lifting device is extended or shortened in a direction perpendicular to the bottom surface.

8. The medical imaging apparatus according to claim 6, wherein the lifting device is positioned between the plurality of link portions.

9. The medical imaging apparatus according to claim 6, wherein the plurality of link portions comprises:
a first link having one side mounted on the bottom surface of the fixing portion and the other side mounted on the base; and
a second link having one side mounted on the base and the other side mounted on the bottom surface of the fixing portion.

10. The medical imaging apparatus according to claim 9, wherein the first link and the second link are pivotably coupled to a central shaft, the first link is pivotably provided with respect to the fixing portion, and the second link is pivotably provided with respect to the base.

11. The medical imaging apparatus according to claim 9, wherein a first rail is provided on the bottom surface of the fixing portion to guide one side of the first link to slide, and a second rail is provided on the base to guide one side of the second link to slide.

12. The medical imaging apparatus according to claim 1, wherein the main body is provided with an opening, and the transport portion slides into the main body through the opening.

13. The medical imaging apparatus according to claim 12, wherein a weight detection sensor for detecting the weight of the patient positioned on the transport portion is provided between the lifting device and the fixing portion.

14. The medical imaging apparatus according to claim 13, wherein the main body is tilted according to information detected by the weight detection sensor.
